(19) Europäisches Patentamt
European Patent Office
Office européen des brevets

(11) EP 4 349 844 A1

(12) EUROPEAN PATENT APPLICATION
published in accordance with Art. 153(4) EPC

(43) Date of publication:
**10.04.2024 Bulletin 2024/15**

(21) Application number: **22815624.6**

(22) Date of filing: **14.03.2022**

(51) International Patent Classification (IPC):
***C07H 15/203*** (2006.01) ***A23L 33/105*** (2016.01)
***A61K 8/60*** (2006.01) ***A61K 31/7034*** (2006.01)
***A61K 36/48*** (2006.01) ***A61P 17/00*** (2006.01)
***A61P 17/18*** (2006.01) ***A61Q 19/00*** (2006.01)
***A61Q 19/02*** (2006.01)

(52) Cooperative Patent Classification (CPC):
**A23L 11/00; A23L 11/60; A23L 33/105; A61K 8/60; A61K 31/7034; A61K 36/48; A61P 17/00; A61P 17/18; A61Q 19/00; A61Q 19/02; C07H 15/203**

(86) International application number:
**PCT/JP2022/011172**

(87) International publication number:
**WO 2022/254867 (08.12.2022 Gazette 2022/49)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **03.06.2021 JP 2021093326**

(71) Applicant: **Nabocul Cosmetics Co., Ltd.**
**Tokyo 101-0051 (JP)**

(72) Inventors:
- **TSIM, Karl Wah-Keung**
  **Hong Kong (CN)**
- **SAKAI, Takako**
  **Tokyo 101-0051 (JP)**
- **DONG, Tina Ting-Xia**
  **Hong Kong (CN)**
- **PENG, Zhi-Tian**
  **Shenzhen (CN)**
- **WANG, Huai-You**
  **Shenzhen (CN)**
- **WEI, James**
  **Tokyo 101-0051 (JP)**

(74) Representative: **Vossius & Partner**
**Patentanwälte Rechtsanwälte mbB**
**Siebertstrasse 3**
**81675 München (DE)**

# (54) NOVEL PHENYLPROPANOID COMPOUND

(57) Provided is a novel active ingredient derived from locust bean and the use thereof. The present invention is a compound represented by the following formula (I) or a salt thereof, or a solvate thereof.

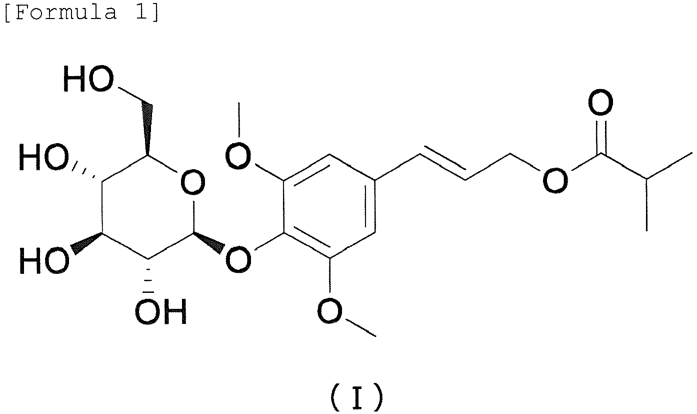

EP 4 349 844 A1

[Formula 1]

(I)

## Description

### Technical Field

The present invention relates to a novel phenylpropanoid compound obtained by separation and purification from locust bean (*Ceratonia siliqua* L.) and the use of the compound as well as a method for producing the compound.

### Background Art

Locust bean (*Ceratonia siliqua L.*) is a leguminous plant mainly native to the Mediterranean region. The pod, that is, a shell and fruits, of locust bean is referred to as a carob and has been long used as food or a food ingredient. A mature pod has a length of about 10 to 25 cm and a sweet taste. The pod of locust bean contains a large amount of polysaccharides, cellulose and minerals, and a small amount of proteins and non-carbohydrate-based low-molecular compounds, and the like. In recent years, research on new functions of locust bean has been in progress, and it has been reported that an aqueous extract of pods of locust bean has multiple pharmacological actions such as antidiarrheal, antioxidant, antimicrobial, antiulcer and anti-inflammatory actions in the gastrointestinal tract, and that it has preventive and therapeutic effects on gastrointestinal diseases such as ulcerative colitis and gastric ulcer (Non Patent Literatures 1 and 2). In addition, Patent Literature 1 discloses that a seed extract of locust bean has an $\alpha$-glucosidase inhibitory activity and thereby has the effect of suppressing the body weight gain (Patent Literature 1).

In the meantime, a free radical is an intermediate product generated during a metabolic process in the body, and are an atom, a group of atoms or a molecule that contains an unpaired electron and have very active chemical properties. Excessive free radicals generated in the body damage the cell membranes of normal cells, denature proteins, and cause loss of the enzymatic functions and damage to DNA and the extracellular matrix. The damage caused by the free radicals is closely related to aging, carcinogenesis, Alzheimer's disease, cardiovascular and cerebrovascular diseases, and skin diseases, and the like. Although the human body has an antioxidant mechanism to prevent the damage caused by free radicals, it is difficult to completely eliminate the damage caused by excess free radicals due to ongoing aging and/or pathological conditions, and the like. Therefore, scavenging free radicals with an antioxidant is very effective for maintaining normal functions of the human body. Examples of the antioxidant currently in use include dibutylhydroxytoluene (BHT) and butylhydroxyanisole (BHA). However, these are expensive and have problems such as poor safety.

When the strong oxidizing power of free radicals damages the extracellular matrix or the like, the skin may lose its elasticity. In addition, due to aging, the skin becomes darker as the skin sags, the texture becomes coarse, and the skin becomes dull. Since the color of human skin mainly depends on the content of melanin produced in the body, the accumulation of melanin in the skin is also one of the important causes for which the skin becomes darker in color and loses its elasticity and luster.

Recent research results show that substances with an antioxidant activity can scavenge free radicals in the body and reduce damage caused by free radicals, so that the substance can slow down the aging process of the skin and, as a result, exert a whitening effect. This suggests that the substances that inhibit melanogenesis and have an antioxidant activity can exert a synergistic whitening effect.

### Citation List

#### Patent Literature

Patent Literature 1
Japanese Patent Laid-Open No. 2005-119999

#### Non Patent Literature

Non Patent Literature 1
Rtibi K, Jabri M A, Selmi S, et al., "Preventive effect of carob (Ceratonia siliqua L.) in dextran sulfate sodium-induced ulcerative colitis in rat", RSC Advances, 2016, Vol. 6, p.19992-20000.
Non Patent Literature 2
Rtibi K, Selmi S, Grami D, et al., "Chemical constituents and pharmacological actions of carob pods and leaves (Ceratonia siliqua L.) on the gastrointestinal tract: A review", Biomedicine & Pharmacotherapy, 2017, Vol. 93, p.522-528.

## Summary of Invention

### Technical Problem

However, Non Patent Literatures 1 and 2 report that the extract of pods of locust bean has pharmacological actions such as antidiarrheal, antioxidant, antimicrobial, antiulcer and anti-inflammatory actions in the gastrointestinal tract and it has therapeutic effects on gastrointestinal diseases, and Patent literature 1 reports that the seed extract of locust bean has the effect of suppressing the body weight gain. However, a specific active ingredient thereof has not yet been identified.

The use of locust bean as a skin-whitening agent has not been previously studied, and thus its effectiveness has been completely unclear.

Accordingly, the present invention has been made in view of the above points. An object of the present invention is to provide a novel active ingredient derived from locust bean and the use thereof.

Another object of the present invention is to provide a novel whitening agent, which is derived from a natural product, locust bean, having both a melanogenesis inhibiting action and an antioxidant activity. A further object of the present invention is to provide a skin external preparation, a cosmetic and a food and drink composition for skin whitening, comprising this whitening agent.

### Solution to Problem

The present inventors have separated a novel phenylpropanoid compound from an extract of pods of locust bean, and have found that this novel compound has not only a melanogenesis inhibiting action but also an antioxidant activity. Based on these findings, the present inventors have completed the present invention.

In order to solve the above problems, the present invention is a compound represented by the following formula (I) or a salt thereof, or a solvate thereof.

[Formula 1]

(I)

The compound represented by formula (I) is a novel phenylpropanoid compound obtained by separation and purification from the extract of pods of locust bean (*Ceratonia siliqua*), and has a melanogenesis inhibiting action and an antioxidant activity as well as a whitening effect.

The whitening agent of the present invention comprises a compound represented by the above formula (I) or a salt thereof, or a solvate thereof. Administration of the compound represented by formula (I) inhibits melanogenesis in the skin as well as scavenges free radicals to inhibit aging of the skin, so that the compound can exert a synergistic whitening effect.

In the whitening agent of the present invention, the concentration of the compound represented by the above formula (I) or a salt thereof, or a solvate thereof is preferably 0.001 mM to 1 mM. This enables the concentration of the active ingredient, having an excellent effect, to be selected.

The melanogenesis inhibitor of the present invention comprises a compound represented by the above formula (I) or a salt thereof, or a solvate thereof. Administration of the compound represented by the formula (I) inhibits melanogenesis in the skin.

The antioxidant agent of the present invention comprises a compound represented by the above formula (I) or a salt thereof, or a solvate thereof. Administration of the compound represented by formula (I) scavenges free radicals to reduce the damage caused by the free radicals.

The whitening agent, melanogenesis inhibitor or antioxidant agent of the present invention is preferably a skin external preparation or cosmetic. Thereby, it is possible to obtain a skin external preparation or cosmetic having a whitening effect, a melanogenesis inhibiting effect or an anti-aging effect on the skin.

The food and drink composition for skin whitening of the present invention comprises a compound represented by the above formula (I) or a salt thereof, or a solvate thereof. Thereby, it is possible to obtain a food and drink composition for skin whitening that inhibits melanogenesis in the skin as well as scavenges free radicals in the body to slow down the aging process of the skin, and as a result, exerts a synergistic whitening effect.

A method for producing a compound represented by the above formula (I) comprises: obtaining a hydroalcoholic extract of pods of locust bean (*Ceratonia siliqua*); subjecting the hydroalcoholic extract to liquid-liquid extraction with petroleum ether and ethyl acetate in this order to collect ethyl acetate fractions; and separating the compound represented by formula (I) from the collected ethyl acetate fractions. Thereby, it is possible to obtain a novel phenylpropanoid compound having an antioxidant activity, a melanogenesis inhibiting action as well as a whitening effect.

Advantageous Effects of Invention

The present invention can provide a novel phenylpropanoid compound as well as a whitening agent, a melanogenesis inhibitor, an antioxidant agent, a skin external preparation, a cosmetic and a food and drink composition for skin whitening comprising the compound, having the following excellent effects.

(1) The phenylpropanoid compound has a melanogenesis inhibiting action and an antioxidant activity as well as a synergistic whitening effect.
(2) They are highly safe for the human body, because they have, as an active ingredient, a compound derived from pods of locust bean that have been edible since ancient times.

Brief Description of Drawings

[Figure 1] Figure 1 is a diagram showing a high-resolution ESI mass spectrum of the compound of the present invention.
[Figure 2] Figure 2 is a diagram showing an ultraviolet absorption spectrum of the compound of the present invention.
[Figure 3] Figure 3 is a diagram showing an infrared absorption spectrum of the compound of the present invention.
[Figure 4] Figure 4 is a diagram showing a $^{1}$H-NMR spectrum ($CD_3OD$, 600 MHz) of the compound of the present invention.
[Figure 5] Figure 5 is a diagram showing a $^{13}$C-NMR spectrum ($CD_3OD$, 150 MHz) of the compound of the present invention.
[Figure 6] Figure 6 is a diagram showing a HSQC spectrum of the compound of the present invention.
[Figure 7] Figure 7 is a diagram showing a HMBC spectrum of the compound of the present invention.
[Figure 8] Figure 8 is a summary of $^{1}$H-NMR signals and $^{13}$C-NMR signals of the compound of the present invention.
[Figure 9] Figure 9 is a diagram showing the HMBC correlation in the compound of the present invention.
[Figure 10] Figure 10 is a graph showing the DPPH radical scavenging activity of the compound of the present invention in Example 4.
[Figure 11] Figure 11 is a graph showing the results of a cytotoxicity test of the compound of the present invention on HepG2 cells in Example 5.
[Figure 12] Figure 12 is a graph showing the ROS generation inhibiting effect of the compound of the present invention on HepG2 cells in Example 5.
[Figure 13] Figure 13 is a graph showing the results of a cytotoxicity test of the compound of the present invention on B16 melanoma cells in Example 6.

Description of Embodiments

Hereinafter, the following will be described: a novel phenylpropanoid compound of the present invention; a whitening agent, a melanogenesis inhibitor, an antioxidant agent, a skin external preparation, a cosmetic and a food and drink composition for skin whitening comprising the compound; and a method for producing the compound.

The novel phenylpropanoid compound represented by the following formula (I) according to the present invention

is a compound having each of glucose and 2-methylpropionic acid linked to sinapyl alcohol by an ester bond.

[Formula 2]

(I)

The novel phenylpropanoid compound according to the present invention may be a salt, and is preferably a pharmacologically acceptable salt. The pharmacologically acceptable salt of the novel phenylpropanoid compound may be any salt formed together with an acid or a base without limitation. The novel phenylpropanoid compound or a salt thereof may be any solvate thereof. Examples thereof include, but are not particularly limited to, a hydrate and an organic solvate such as a solvate with ethanol.

The novel phenylpropanoid compound according to the present invention has an excellent melanogenesis inhibiting action and also an antioxidant action, and can be therefore used as a whitening agent that provides a whitening effect through multiple mechanisms. That is, the novel phenylpropanoid compound according to the present invention inhibits melanogenesis in the skin as well as scavenges free radicals by its antioxidant action to inhibit darkening of the skin due to skin aging resulting from the damage caused by free radicals, so that it can exert a synergistic whitening effect.

In the context of the present invention, the term "melanogenesis inhibiting action" means an action of reducing the amount of melanin produced in melanocytes such as B16 melanoma cells as compared to a control without addition or administration of the novel phenylpropanoid compound of the present invention. More specifically, the amount of melanin produced is preferably 80% or less, more preferably 70% or less of the control.

In the context of the present invention, the term "antioxidant action" means, for example, a higher rate of DPPH radical scavenging or an action of reducing intracellular ROS compared to a control without addition or administration of the novel phenylpropanoid compound of the present invention.

The novel phenylpropanoid compound of the present invention can be obtained by separation and purification from pods of locust bean. The locust bean used in the present invention has a scientific name of *Ceratonia siliqua,* and is a plant belonging to the family Fabaceae, the subfamily Caesalpinioideae, the genus Ceratonia. It is a plant mainly native to the Mediterranean region, but in the present invention, the production area and cultivation environment are not particularly limited and locust bean from any production area and cultivation environment can be used.

A method for separating the novel phenylpropanoid compound of the present invention from locust bean will be described. First, a hydroalcoholic extract is obtained from pods of locust bean. In the context of the present invention, "hydroalcoholic extract of pods of locust bean" refers to an extract obtained by subjecting pods of locust bean to an extraction treatment by adding a hydrous alcohol as an extraction solvent. The pod of locust bean refers to a shell and fruits of "fruits with a shell of locust bean", and either one of the shell or the fruits can be used as an extraction material, but both the shell and the fruits are more preferably used. The extraction treatment is subjected to the as-harvested, i.e., fresh or dried pods of locust bean. However, the extraction treatment can be subjected to the pods of locust bean that have been subjected to various pretreatment in order to improve extraction efficiency or facilitate handling. Examples of the pretreatment include, but are not particularly limited to, drying treatment, crushing treatment and grinding treatment. The pods of locust bean that have been subjected to such a pretreatment may be also subjected to the extraction treatment to obtain an extract.

The alcohol constituting the hydrous alcohol used as an extraction solvent is not particularly limited as long as it can extract the phenylpropanoid compound of the present invention. Examples of such an alcohol include ethanol, methanol, propanol, isopropanol, butanol and isobutanol. Among these, hydrous ethanol is preferably selected as an extraction solvent from the viewpoint of safety to the human body, extraction efficiency, and the like. The concentration

of the hydrous alcohol is preferably 50 to 99%, more preferably 60 to 97%, and particularly preferably 70 to 95%. The extraction solvent can further comprise other ingredients unless they interfere with the extraction of the compound of the present invention.

The method of extracting with a hydrous alcohol is a method comprising extracting pods of locust bean by adding the hydrous alcohol as an extraction solvent to the pods and immersing the pods in the hydrous alcohol. For example, when the pods of locust bean are dry crushed material with a water content of less than 10%, the extraction solvent is preferably used in the amount of 5 to 10 parts by weight per 1 part by weight of the plant body. The extraction method may be any method such as extraction at room temperature, extraction by heating, extraction by pressurizing and heating, or subcritical extraction, but extraction by heating under reflux is preferable from the viewpoint of extraction efficiency. In order to increase the extraction efficiency, the extraction operation is preferably performed multiple times, and more preferably performed multiple times with different alcohol concentrations in the extraction solvent. Examples of the extraction method include, but are not particularly limited to, an extraction method comprising performing extraction under reflux with 95% ethanol one to five times followed by extraction under reflux with 70% ethanol one to five times. The extraction time is variously set, depending on the extraction method, the type of extraction material, the type of extraction solvent or the extraction temperature or the like. However, for example, when the extraction under reflux is performed with 70 to 95% ethanol, the extraction time for one extraction is preferably set to about 1 to 3 hours, and particularly preferably about 1.5 hours. After the extraction treatment described above, the residue is removed by decantation, centrifugation or filtration, or the like to obtain a hydroalcoholic extract of pods of locust bean. The obtained extract also includes a concentrate or a solid obtained by subjecting the extract to a treatment such as distillation under reduced pressure.

The hydroalcoholic extract of pods of locust bean obtained as described above may contain saccharides and the like that are contained in large amounts in the pods of locust bean. Therefore, for the purpose of removing these unnecessary components, the hydroalcoholic extract may be subjected to separation operation with an ion exchange resin. Specifically, 1 to 10 parts by weight of water is added to 1 part by weight of a hydroalcoholic extract of pods of locust bean and disperse the extract in water. The dispersion is then passed through a column packed with an ion exchange resin such as a macroporous adsorption resin to adsorb the phenylpropanoid compound of the present invention and flow out and remove unnecessary components such as saccharides. Thereafter, the adsorbed phenylpropanoid compound of the present invention is eluted with 95% ethanol or the like to collect fractions containing the phenylpropanoid compound of the present invention.

Next, a further separation operation to which the hydroalcoholic extract of pods of locust bean or the collected fractions separated by the ion exchange resin described above are subjected will be described. The hydroalcoholic extract or the collected fractions are dispersed in an aqueous solvent and are subjected to solvent extraction with petroleum ether and ethyl acetate in this order. The aqueous solvent is not particularly limited as long as it can disperse the phenylpropanoid compound of the present invention, but a 50% hydrous methanol is suitably used. After performing liquid-liquid extraction with petroleum ether/an aqueous solvent multiple times, liquid-liquid extraction with ethyl acetate/an aqueous solvent is performed multiple times. The ethyl acetate fractions collected by this solvent extraction operation contain the novel phenylpropanoid compound of the present invention. The number of liquid-liquid extractions in each solvent system is preferably about 2 to 10, particularly preferably about 5.

The methyl acetate fractions obtained as described above can be purified by a conventional method to isolate the novel phenylpropanoid compound of the present invention. Examples of the purification method includes normal phase chromatography, reverse phase chromatography, thin layer chromatography, gel filtration chromatography and high-performance liquid chromatography. Purification may be performed by using one of these chromatographies or two or more in combination. Carriers, elution solvents and the like used in each chromatography can be appropriately selected depending on the chromatography.

The phenylpropanoid compound of the present invention which has been separated and purified from pods of locus bean as described above may not be isolated as a completely pure substance, and may contain some other components derived from a locust bean raw material.

The novel phenylpropanoid compound of the present invention may be a synthetic product synthetically obtained by any known method.

The novel phenylpropanoid compound of the present invention has an excellent melanogenesis inhibiting action and also an antioxidant action, and can be therefore used as a whitening agent that provides a whitening effect through multiple mechanisms. The phenylpropanoid compound can be also used as a melanogenesis inhibitor or an antioxidant agent.

The whitening agent and melanogenesis inhibitor comprising the novel phenylpropanoid compound of the present invention can be used as a skin external preparation or a cosmetic for inhibiting melanogenesis in the skin as well as inhibiting darkening of the skin due to skin aging resulting from the damage caused by free radicals. The antioxidant agent comprising the novel phenylpropanoid compound of the present invention can be also used as a skin external preparation for preventing skin aging resulting from the damage caused by free radicals and keeping the skin in a stable

condition. The antioxidant agent of the present invention can be also used as a cosmetic that has the effect of preventing skin aging resulting from the damage caused by free radicals, preventing sagging of the skin, coarsening of texture of the skin and dullness of the skin and keeping the skin in a healthy condition.

The dosage of the whitening agent, melanogenesis inhibitor and antioxidant agent of the present invention varies depending on the target effect, therapeutic effect, administration method or age, or the like, and cannot be determined in general. However, when used as an external preparation, the usual daily parenteral dosage is preferably 0.2 μg to 30 mg, more preferably 1 μg to 3 mg, and even more preferably 5 μg to 500 μg of the phenylpropanoid compound of the present invention. When used as an internal preparation, the usual daily oral dosage is preferably 1 μg to 1000 mg, and more preferably 5 μg to 500 mg of the phenylpropanoid compound of the present invention.

The dosage form of the whitening agent, melanogenesis inhibitor and antioxidant agent as well as the skin external preparation and the cosmetic of the present invention is not particularly limited, and examples thereof include liquids such as low-viscosity liquids or lotions; emulsions, gels, pastes, creams, foams, packs, ointments, powders, aerosols and patches; and tablets, granules, capsules and liquids for internal application. The whitening agent, melanogenesis inhibitor and antioxidant agent according to the present invention can be applied for any of a cosmetic, a quasi-drug and a pharmaceutical. Specific examples of the cosmetic, quasi-drug and pharmaceutical include, but are not particularly limited to, cosmetic lotions, cosmetic creams, cosmetic emulsions, serums, cosmetic packs, cosmetic cleansers, soaps, hair care agents, bath agents, makeup cosmetics, and anti-acne skin care cosmetics.

The blending concentration of the novel phenylpropanoid compound of the present invention contained in the whitening agent, melanogenesis inhibitor and antioxidant agent as well as the skin external preparation and cosmetic is preferably 0.001 mM to 1 mM, more preferably 5 μM to 100 μM, and even more preferably 10 μM to 100 μM. When the blending amount of the novel phenylpropanoid compound of the present invention is within this range, the compound can be stably blended, it can be highly safe for the skin and it can exert a high whitening effect, melanogenesis inhibiting action and antioxidant action.

The whitening agent, melanogenesis inhibitor and antioxidant agent of the present invention can be prepared in various forms by any method conventionally used. In this case, it can be formulated with additives that are usually accepted as pharmaceutical additives, such as carriers and excipients for formulations. In addition, in order to improve the bioavailability and stability of the present compound, a drug delivery system including a formulation technique such as microencapsulation, pulverization or inclusion with cyclodextrin or the like can be also used.

The whitening agent, melanogenesis inhibitor and antioxidant agent as well as the skin external preparation and the cosmetic of the present invention can comprise appropriately an ingredients commonly used in an external skin preparations and cosmetics, such as water, fats and oils, waxes, hydrocarbons, fatty acids, higher alcohols, esters, plant extracts, vitamins, water-soluble polymers, surfactants, metallic soaps, alcohols, polyhydric alcohols, pH adjustors, preservatives, fragrances, powders, thickeners, pigments and chelating agents. In addition, one or two or more of various functional ingredients usually used, for example, selected from moisturizing agents, anti-inflammatory agents, cell activators, ultraviolet protection agents, blood circulation promoters and other whitening agents/antioxidants and the like can be used in combination with the compound of the present invention, unless they impair the actions and effects of the present invention.

The food and drink composition for skin whitening of the present invention also comprises, as an active ingredient, the novel phenylpropanoid compound of the present invention. The food and drink composition for skin whitening of the present invention can be prepared in any form, for example, in the form of supplements such as tablets, capsules, granules and syrups; drinks such as soft drinks, fruit juice drinks, and alcoholic drinks; and sweets such as candies, gums, cookies, biscuits and chocolates; breads, porridges, cereals, noodles, jellies, soups, dairy products and seasonings. When used as food or drink products in this way, the food and drink composition for skin whitening can be combined in various manner with various other active ingredients, or nutrients such as vitamins, minerals and amino acids unless they affect the efficacy of the active ingredient of the present invention. The food and drink product of the present invention include supplements, health foods, functional foods, foods for specified health uses, and the like. The daily intake of the food and drink product of the present invention is preferably 1 μg to 1000 mg and more preferably 5 μg to 500 mg of the phenylpropanoid compound of the present invention.

Hereinafter, the present invention will be described in detail with reference to examples thereof, but the present invention will not be limited by these examples.

## Examples

### [Example 1]

1. Preparation of hydroalcoholic extract of pods of locust bean.

Seeds were removed from fruits with shells of locust bean (*Ceratonia siliqua*) that had been subjected to drying

treatment after harvesting. Pods of this locust bean was ground with a grinder to obtain a ground product having a particle size of 2 mm or less. The operation of adding, to 20 kg of the ground product, 140 kg (7 times the amount of the ground product) of a 95% hydrous ethanol and extracting under refluxing for 1.5 hours was performed twice. Thereafter, the residue was further extracted under reflux with 140 kg (7 times the amount of the ground product) of a 70% hydrous ethanol for 1.5 hours. The extracts obtained by extraction under reflux were combined, and the solvent was then distilled off under reduced pressure to obtain 12.4 kg of a hydrous ethanol extract of pods of locust bean.

[Example 2]

2. Separation and purification of hydroalcoholic extract of pods of locust bean

The hydroalcoholic extract of pods of locust bean obtained in Example 1 was dispersed in 1 to 10 times the amount of water and adsorbed on an ion exchange resin (a macroporous adsorption resin D101, available from Cangzhou Bon Adsorber Technology Co., Ltd.). Impurities such as saccharides were removed by eluting them with 3 times the column volume of distilled water, elution was then performed with 3 times the column volume of a 95% hydrous ethanol, and the solvent was distilled off under reduced pressure to obtain 462.7 g of ethanol elution fractions (non-carbohydrate-based low molecular compound fractions). Next, the obtained ethanol elution fractions were dispersed in 1.0 L of 50% hydrous methanol, and the dispersion was subjected to liquid-liquid extraction with petroleum ether and ethyl acetate in this order each five times. Each solvent was distilled off under reduced pressure to obtain 28.4 g of petroleum ether fractions, 139.4 g of ethyl acetate fractions and 290.2 g of aqueous fractions, respectively.

Then, a 135.0g portion of the ethyl acetate fractions was subjected to normal phase silica gel column chromatography (a column packing material: 200 to 300 mesh; a product available from Qingdao Haiyang Chemical Co., Ltd.), and elution and fractionation were performed with two developing solvents, that is, petroleum ether (P)/ethyl acetate (E) and dichloromethane (C)/methanol (M). As a result, 108 elution fractions were obtained. The obtained 108 elution fractions were subjected to identification with thin layer chromatography (a silica gel $GF_{254}$ plate; a product available from Qingdao Haiyang Chemical Co., Ltd.), and similar fractions were combined to obtain ten elution fractions A to J.

Then, the elution fraction I (20.0 g) was subjected to reverse phase ODS column chromatography (a column packing material: 40 to 63 μm; a product available from Merck & Co.), and fractionation was performed by gradient elution with methanol: water = 15:85 → 100:0. The obtained elution fractions were subjected to identification with thin layer chromatography (a silica gel $GF_{254}$ plate; a product available from Qingdao Haiyang Chemical Co., Ltd.), and similar fractions were combined to obtain five elution fractions I1 to I5.

Next, the elution fraction I5 (2.7 g) was subjected to gel filtration chromatography (a column packing material: Sephadex LH-20), and elution was performed with dichloromethane: methanol = 1:1 to obtain five elution fractions 15a to I5e.

Then, the elution fraction I5b (0.5 g) was subjected to semi-preparative HPLC (Column I: YMC-Pack ODS-A, 250 × 20 mm, 5 μm), and separation was performed with methanol:water = 55:45 at a detection wavelength of 220 nm to obtain a fraction I5b1. This fraction I5b1 was subjected to semi-preparative HPLC (Column II: YMC-Pack ODS-A, 250 × 10 mm, 5 μm), and separation was performed with methanol:water = 55:45 at a detection wavelength of 267 nm to obtain 48.3 mg of a compound of the present invention (hereinafter referred to as "Ceratonia siliqua B") (retention time tR = 27.27 minutes).

[Example 3]

3. Structural analysis of compound (Ceratonia siliqua B)

The compound obtained in Example 2, "Ceratonia siliqua B" was subjected to structural analysis. For structural analysis, high-resolution mass spectrometry (HR-ESI-MS; positive ion mode), ultraviolet absorption spectrometry, infrared absorption spectrometry and $^{1}H$-NMR, $^{13}C$-NMR, HMBC and HSQC analyses were performed. The following devices were used for these analyses.

- High-resolution mass spectrometry: an electrospray ionization quadrupole time-of-flight mass spectrometry device (a product available from Bruker Daltonics)
- Ultraviolet absorption spectrometry: an ultraviolet-visible spectrophotometer (UV-2401PC, a product available from Shimadzu Corporation)
- Infrared absorption spectrometry: FT-IR (NEXUS470, a product available from Thermo nicolet)
- NMR analysis: a 600 MHz nuclear magnetic resonance device (AVANCE III 600, a product available from Bruker)

The physical properties of Ceratonia siliqua B are as follows. The spectrum of high-resolution mass spectrometry

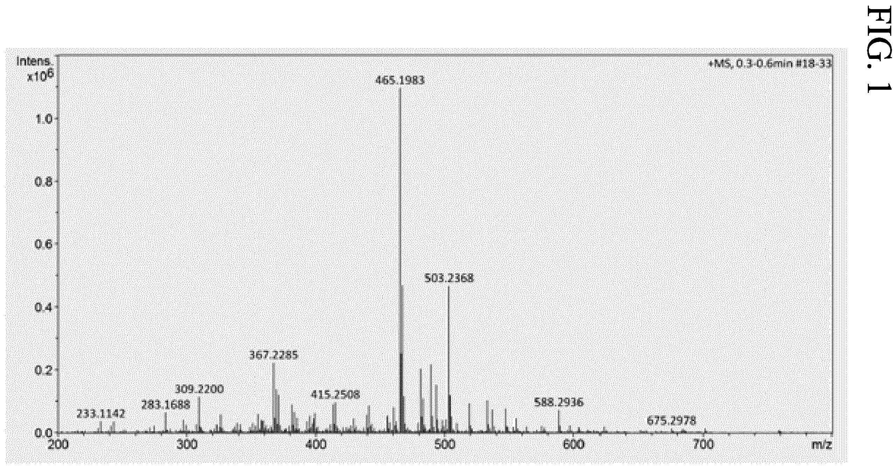

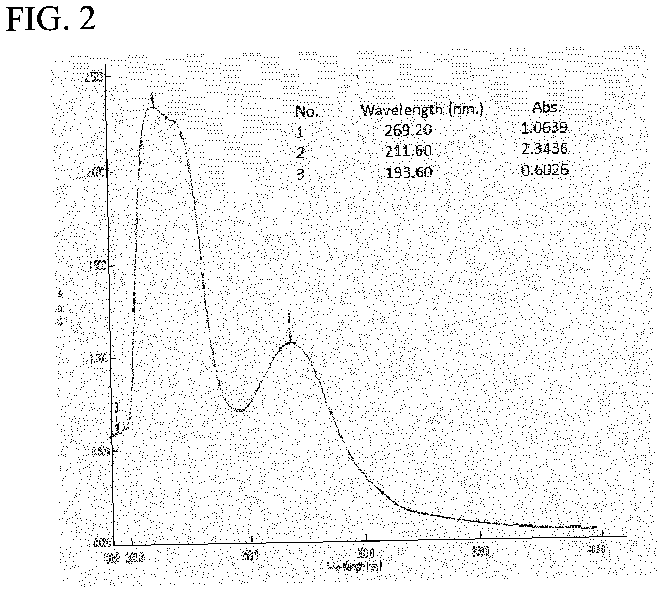

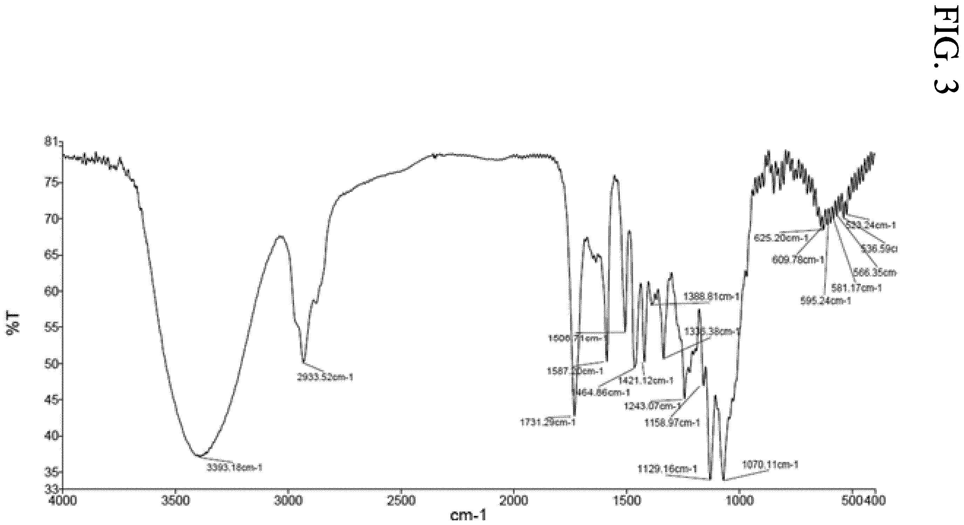

(HR-ESI-MS) is shown in Figure 1, the results of ultraviolet absorption spectrometry are shown in Figure 2, and the results of infrared absorption spectrometry are shown in Figure 3.

- Aspect: yellow powder
- HR-ESI-MS (positive) m/z: 465.1983 $[M + Na]^+$
- Ultraviolet absorption spectrum: λmax (MeOH) nm (log ε): 193.6 (3.73), 211.6 (4.32), 269.2 (3.97)
- Infrared absorption spectrum (KBr, $cm^{-1}$): vmax: 3393.18, 2933.52, 1731.29, 1587.20, 1506.71, 1464.86, 1421.12, 1388.81, 1336.38, 1243.07, 1158.97, 1129.16, 1070.11, 625.20, 609.78, 595.24, 581.17, 566.35, 536.59, 523.24

The results of high-resolution mass spectrometry (Figure 1) showed that the quasi-molecular ion peak was m/z 465.1983 $[M + Na]^+$, so that the compositional formula was estimated to be $C_{21}H_{30}O_{10}$ and the degree of unsaturation was estimated to be 7. The ultraviolet absorption spectrum (Figure 2) showed maximum absorption at 211 nm and 269 nm, suggesting the possibility that Ceratonia siliqua B would be a phenylpropanoid-based compound. The infrared absorption spectrum (Figure 3) showed that the molecular structure included functional groups such as a hydroxyl group (3393.18 $cm^{-1}$), a benzene ring (1587.20 $cm^{-1}$, 1506.71 $cm^{-1}$) and an ester bond (1731.29 $cm^{-1}$, 1129.16 $cm^{-1}$).

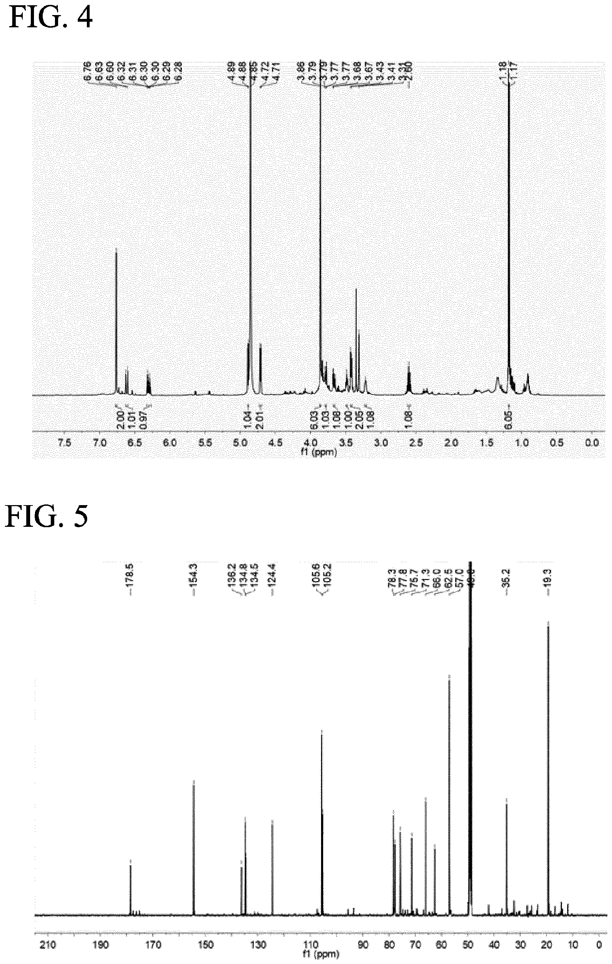

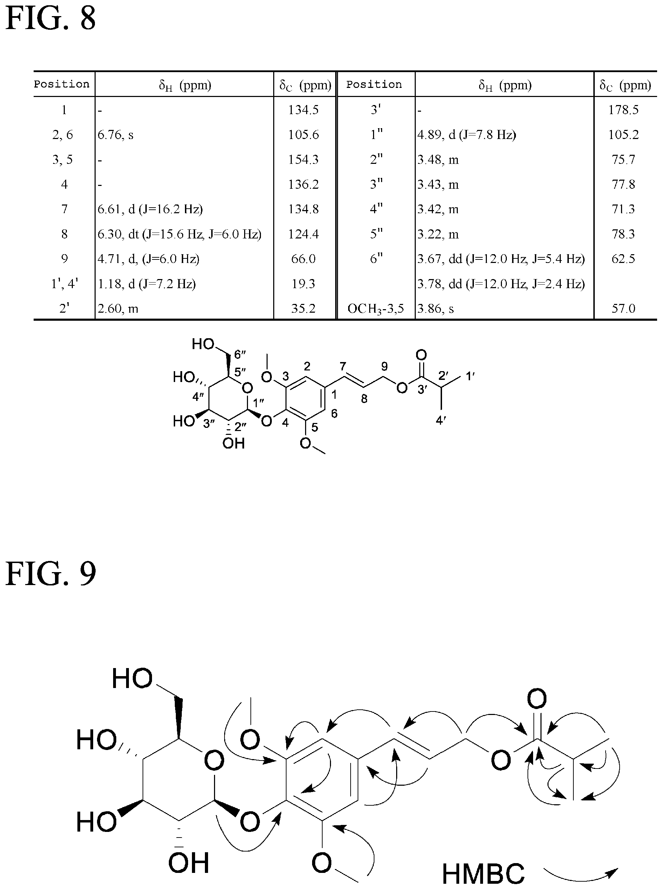

From the spectrum (Figure 4) obtained by $^1$H-NMR analysis (a solvent: $CD_3OD$, observation frequency: 600 MHz), the following was confirmed (Figure 8): a set of 1,3,4,5-substituted benzene ring hydrogen signals [$\delta_H$: 6.76 (2H, s, H-2, H-6)]; a set of trans-olefin proton signals $\delta_H$: [6.61 (1H, d, J = 16.2 Hz, H-7), 6.30 (1H, dt, J = 15.6 Hz, J = 6.0 Hz, H-8)]; one methylene hydrogen signal [$\delta_H$: 4.71 (2H, d, J = 6.0 Hz, H-9)]; two methoxy group signals [$\delta_H$: 3.86 (6H, s, $OCH_3$-3, $OCH_3$-5)]; one methine group signal [$\delta_H$: 2.60 (1H, m, H-2')]; two methyl group signals [$\delta_H$: 1.18 (6H, d, J = 7.2 Hz, $H_3$-1'/$H_3$-4')]; and a set of glucose residue hydrogen signals [$\delta_H$: 4.89 (1H, d, J = 7.8 Hz, H-1"), 3.78 (1H, dd, J = 12.0 Hz, J = 2.4 Hz, H-6α"), 3.67 (1H, dd, J = 12.0 Hz, J = 5.4 Hz, H-6β"), 3.48 (1H, m, H-2"), 3.43 (1H, m, H-3"), 3.42 (1H, m, H-4"), 3.22 (1H, m, H-5")]. From the spectrum (Figure 5) obtained by $^{13}$C-NMR analysis (a solvent: $CD_3OD$, observation frequency: 150 MHz), 21 carbon signals were confirmed (Figure 8). They included the following: one ester carbonyl group carbon signal ($\delta_C$: 178.5); eight aromatic or olefinic carbon signals (δc: 154.3 × 2, 136.2, 134.8, 134.5, 124.4, 105.6 × 2); one methylene group signal (δc: 66.0), two methoxy group signals ($\delta_C$: 57.0 × 2); one methine group signal (δc: 35.2); two methyl group signals (δc: 19.3 × 2); and a set of glucose residue carbon signals (δc: 105.2, 78.3, 77.8, 75.7, 71.3, 62.5). These analysis results showed that the NMR data of this compound is similar to that of syringin, but this compound is different from syringin in that this compound further contains a 2-methylpropionic acid unit.

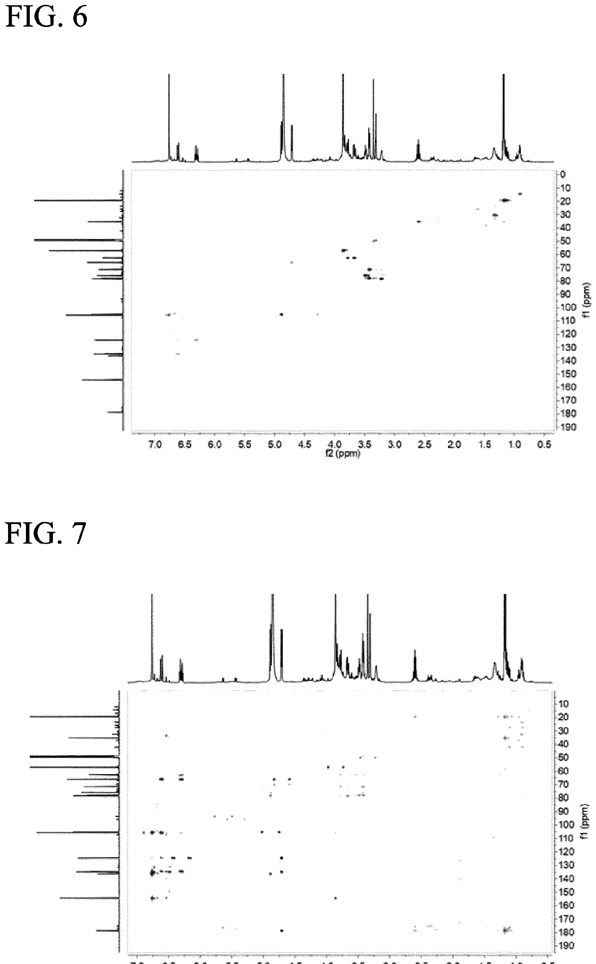

In addition, from the spectrum obtained by HMBC analysis (Figure 7), long-range correlation signals were confirmed between H-2/H-6 and C-4, between H-7 and C-2/C-6, between H-8 and C-1, between H-9 and C-7 and between H-1" and C-4. This result showed the presence of a syringin unit in the structure of this compound. Further, long range correlation signals were confirmed between $H_3$-1' and C-3'/C-4', and between H-2' and C-3'/C-4', indicating the presence of a 2-methylpropionic acid unit in the structure of this compound. Furthermore, long-range correlation signals were confirmed between $H_2$-9 and C-3', indicating that the 2-methylpropionic acid unit was attached at the C-9 position. The coupling constant of the proton at the glucose terminal was 7.8 Hz, indicating that the relative configuration of the glucose terminal was β-type. From these analysis results, the structure of this compound, Ceratonia siliqua B was determined to be represented by the following formula (I). Figure 8 shows an assignment table summarizing $\delta_H$ of $^1$H-NMR signals and $\delta_C$ of $^{13}$C-NMR signals based on HSQC spectrometry and HMBC spectrometry. Figure 9 shows the analysis results of HMBC for Ceratonia siliqua B.

[Formula 3]

(I)

[Example 4]

4. Antioxidant activity of Ceratonia siliqua B (1)

Ceratonia siliqua B obtained in Example 2 was used to measure the DPPH radical scavenging activity of Ceratonia siliqua B.

First, Ceratonia siliqua B obtained in Example 2 was accurately weighed out and dissolved in methanol to prepare a sample solution with a concentration of 100 μM. This sample solution having a concentration of 100 μM was further diluted with methanol to obtain sample solutions with different concentrations of 1 μM, 5 μM, 10 μM and 50 μM. Next, 2 mg of DPPH (2,2-diphenyl-1-picrylhydrazyl) was dissolved in 50 mL of methanol to prepare a 100 μM DPPH solution.

A 190 μL portion of the DPPH solution was placed in each of wells of a 96-well microplate, and 10 μL of the sample solution was quickly added thereto. The total volume of the reaction solution in each well was 200 μL. The plate was gently shaken, and the reaction solution was allowed to react at room temperature for 30 minutes in the dark. Thereafter, the absorbance at 517 nm ($A_{sample}$) was measured with a microplate reader. The reaction solution to which methanol was added instead of the sample solution was used as a blank, and the absorbance of the blank ($A_{blank}$) was measured. In addition, the absorbance ($A_{sample\ blank}$) due to coloration of the sample solution itself at each concentration was measured as a sample blank. The test was performed in triplicate (3 wells) for each concentration of the sample solution, the blank and the sample blank (n = 3).

The radical scavenging rate (%) was determined by the following equation.

$$\text{Radical scavenging rate (\%)} = [A_{blank} - (A_{sample} - A_{sample\ blank})]/A_{blank} \times 100$$

wherein $A_{blank}$ is the absorbance of the blank, $A_{sample}$ is the absorbance of the sample solution, and $A_{sample\ blank}$ is the absorbance of the sample blank.

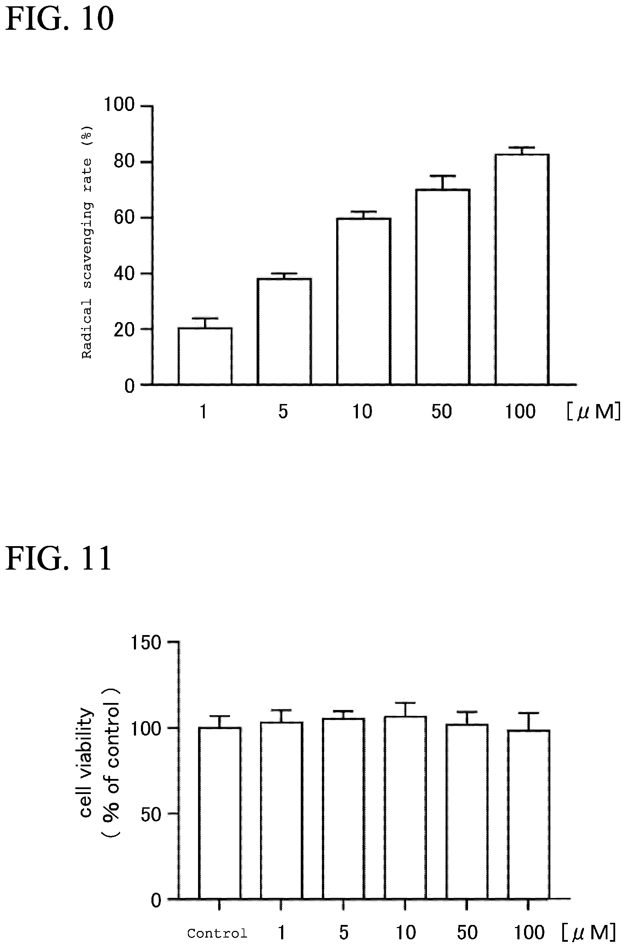

The results are shown in Figure 10. The compound of the present invention, Ceratonia siliqua B, was shown to scavenge DPPH radicals in a concentration-dependent manner. This shows that Ceratonia siliqua B has a radical scavenging activity, that is, an antioxidant activity.

[Example 5]

5. Antioxidant activity of Ceratonia siliqua B (2)

Ceratonia siliqua B obtained in Example 2 was used to examine the effect of Ceratonia siliqua B on the generation of reactive oxygen species (ROS) in living cells.

(a) Living cells and their cell culture conditions

In this test, a HepG2 cell, a human liver-derived tumor cell line was used as a living cell. HepG2 cells were cultured in a DMEM medium (containing 10% FBS, 100 U/mL penicillin and 100 μM/mL streptomycin) in the presence of 5% $CO_2$ at 37°C and saturated humidity.

(b) Preparation of sample solution

Ceratonia siliqua B was diluted with a DMEM medium to obtain sample solutions with different concentrations of 1 μM, 5 μM, 10 μM, 50 μM and 100 μM.

(c) Study about cytotoxicity on HepG2 cells

First, an MTT test was performed to confirm the presence or absence of cytotoxicity of Ceratonia siliqua B against HepG2 cells. HepG2 cells that have been subcultured for three generations or more were treated with trypsin to prepare a single cell suspension having a concentration of $1 \times 10^5$ cells/mL. A 100 μL portion of this cell suspension was seeded in each well of a 96-well microplate and cultured in a $CO_2$ incubator for 24 hours. After culturing, the supernatant was removed, and the wells were divided into a control (blank control) group and a test group. A 200 μL portion of the DMEM medium was added to each well of the control group, and 200 μL of each of the sample solutions

from the above (b) was added to each well of the test group. After culturing in the $CO_2$ incubator for 48 hours, the supernatant was removed, and 100 μL of 0.5 mg/mL MTT was added to each well. After 4 hours, the supernatant was removed, a 150 μL portion of DMSO was added to each well, and the absorbance at 570 nm was measured with a microplate reader. The test was performed in triplicate (3 wells) for the control group and for each concentration of the sample solution in the test group (n = 3).

The results are shown in Figure 11. The cell viability of the test group was calculated taking the value for the control (blank control) group as 100. The compound of the present invention, Ceratonia siliqua B was found to have no cytotoxicity against HepG2 cells at concentrations of 1 to 100 μM.

(d) Measurement of ROS in HepG2 cells

A HepG2 cell suspension having a concentration of $1 \times 10^5$ cells/mL was prepared, and a 100 μL portion of the cell suspension was seeded in each well of a 96-well microplate and cultured in a $CO_2$ incubator for 24 hours. These wells were divided into a blank control group, a positive control group and a test group. A 100 μL portion of a DMEM medium was added to each well of the blank control group; a 100 μL portion of a solution of L-ascorbic acid dissolved in the DMEM medium was added to each well of the positive control group (a final concentration of L-ascorbic acid: 100 μM); and a 100 μL portion of each of solutions obtained by appropriately diluting Ceratonia siliqua B with the DMEM medium was added to each well of the test group to provide a different concentration from each other (a final concentration: 1 μM, 5 μM, 10 μM, 50 μM and 100 μM). After culturing in a $CO_2$ incubator for 24 hours, the culture medium was removed from each well, and 100 μL of a serum-free medium was added thereto followed by washing, which was repeated three times. Next, a 100 μL portion of 10 μM DCFH-DA (2',7'-dichlorofluorescin diacetate) was added to each well and incubated in the $CO_2$ incubator for 60 minutes. Thereafter, DCFH-DA was removed from each well, and a 100 μL portion of the serum-free medium was added thereto followed by washing, which was repeated three times. Finally, 100 μL portion of 400 μM hydrogen peroxide ($H_2O_2$) diluted with the serum-free medium was added to each well and incubated for 90 minutes. Thereafter, the medium in each well was removed, 100 μL of PBS was added thereto, and fluorescence intensity at 535 nm was measured at an excitation wavelength of 485 nm. The amount of ROS generated in the positive control group and the test group was calculated taking the fluorescence intensity of the blank control group as 1.0 (basal). The test was performed in triplicate (3 wells) for the blank control group, for the positive control group, and for each concentration of the sample solution in the test group (n = 3).

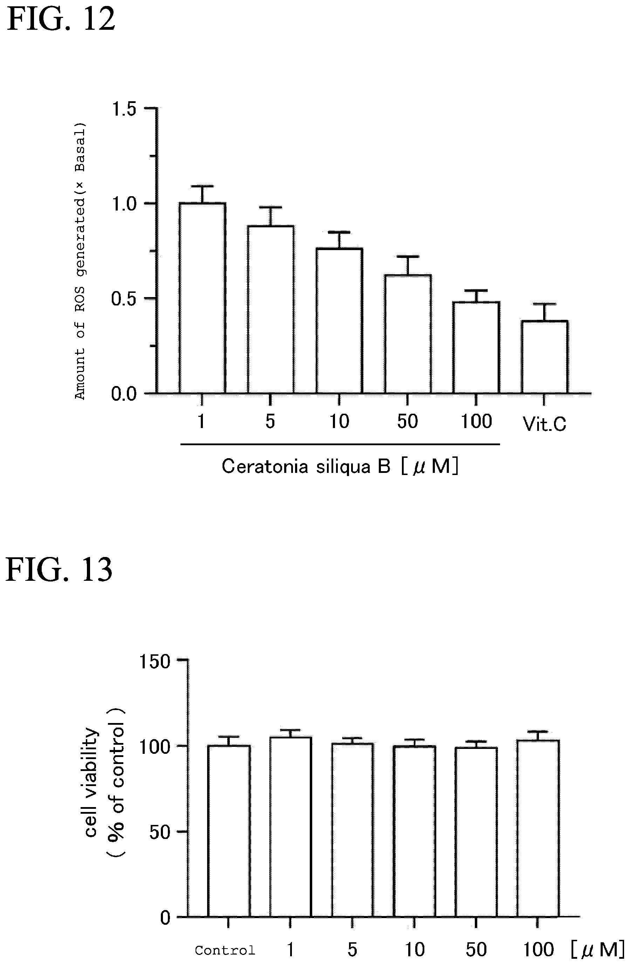

The results are shown in Figure 12. Vit. C represents 100 μM L-ascorbic acid as a positive control. Figure 12 shows that the compound of the present invention, Ceratonia siliqua B, inhibited the increase in ROS induced by hydrogen peroxide in HepG2 cells in a concentration-dependent manner, indicating that Ceratonia siliqua B has an antioxidant activity that reduces the amount of ROS generated in living cells.

[Example 6]

6. Melanogenesis inhibiting effect of Ceratonia siliqua B

Ceratonia siliqua B obtained in Example 2 was used to measure the melanogenesis inhibiting effect of Ceratonia siliqua B.

(a) Cell culture condition

In this test, B16 melanoma cells were used. A B16 melanoma cell, a mouse skin melanoma-derived cell line, has melanogenic capacity. B16 melanoma cells were cultured in a DMEM medium (containing 10% FBS, 100 U/mL penicillin and 100 μM/mL streptomycin) in the presence of 5% $CO_2$ at 37°C and saturated humidity.

(b) Preparation of sample solution

Ceratonia siliqua B was diluted with a DMEM medium to obtain sample solutions with different concentrations of 1 μM, 5 μM, 10 μM, 50 μM and 100 μM.

(c) Study about cytotoxicity against B16 melanoma cells

First, an MTT test was performed to confirm the presence or absence of cytotoxicity of Ceratonia siliqua B against B16 melanoma cells. B16 melanoma cells that had been subcultured for three generations or more were treated with trypsin to prepare a single cell suspension having a concentration of $3 \times 10^4$ cells/mL. A 100 μL portion of this cell suspension was seeded in each well of a 96-well microplate and cultured overnight in a $CO_2$ incubator. The supernatant

was removed from each well, and the wells were divided into a control (blank control) group and a test group. A 200 μL portion of a DMEM medium was added to the control group, and 200 μL of each of the sample solutions from the above (b) was added to each well of the test group. After culturing in the $CO_2$ incubator for 48 hours, the supernatant was removed and a 100 μL portion of 0.5 mg/mL MTT was added to each well. After 4 hours, the supernatant was removed, a 150 μL portion of DMSO was added to each well, and the absorbance at 570 nm was measured with a microplate reader. The test was performed in triplicate (3 wells) for the control group and for each concentration of the sample solution in the test group (n = 3).

The results are shown in Figure 13. The cell viability of the test group was calculated taking the value for the control (blank control) group as 100. The compound of the present invention, Ceratonia siliqua B, was found to have no cytotoxicity against B16 melanoma cells at concentrations of 1 to 100 μM.

(d) Measurement of amount of melanin produced in B16 melanoma cells

A single cell suspension of B16 melanoma cells was prepared, and $4 \times 10^4$ B16 melanoma cells were seeded in each well of a 12-well microplate. After culturing overnight in a $CO_2$ incubator, the supernatant was removed, and the wells were divided into a blank control group, a positive control group and a test group. A 1 mL portion of a DMEM medium was added to each well of the blank control group, a 1 mL portion of each of solutions of L-ascorbic acid dissolved in DMEM medium at different concentrations (a concentration of L-ascorbic acid: 0.1 μM, 5 μM, 10 μM) was added to each well of the positive control group, and a 1 mL portion of each of the sample solutions from the above (b) was added to each well of the test group. After culturing for 48 hours, the supernatant was removed from each well followed by washing twice with PBS buffer, treatment with trypsin and addition of 1.2 mL of PBS buffer to prepare a single cell suspension. A 200 μL aliquot of this cell suspension was taken to determine the protein content. The remaining cell suspension was centrifuged at 3000 rpm for 5 minutes to remove the supernatant. Thereafter, 250 μL of a 1 M NaOH solution containing 1% DMSO was added thereto and dissolved for 30 minutes in a water bath heated to 80°C. A 200 μL aliquot of this solution was taken and the absorbance at 405 nm ($A_{test\ group}$, $A_{positive\ control\ group}$, $A_{blank\ control\ group}$) was measured. The test was performed in triplicate (3 wells) for the blank control group, for the positive control group and for each concentration of the sample solution in the test group (n = 3).

The intracellular melanogenesis inhibition rate (%) was determined for the test group and the positive control group by the following equations:

$$\text{Melanogenesis inhibition rate of test group (\%)} = 1 - [(A_{test\ group}/\text{protein content of test group})/(A_{blank\ control\ group}/\text{protein content of blank control group})];$$

and

$$\text{Melanogenesis inhibition rate of positive control group (\%)} = 1 - [(A_{positive\ control\ group}/\text{protein content of positive control group})/(A_{blank\ control\ group}/\text{protein content of blank control group})];$$

wherein $A_{test\ group}$ is the absorbance of the test group, $A_{positive\ control\ group}$ is the absorbance of the positive control group, and $A_{blank\ control\ group}$ is the absorbance of the blank control group.

The results are shown in Table 1 below. L-Ascorbic acid as a positive control exhibited a significant inhibition effect on melanogenesis at a concentration of 10 μM ($P < 0.01$), which corresponded to an inhibition rate of 32.8%. The compound of the present invention, Ceratonia siliqua B, was also found to have a significant inhibition effect on melanogenesis in B16 melanoma cells at a concentration of 10 to 100 μM ($P < 0.05$). The melanogenesis inhibiting effect of Ceratonia siliqua B increased in proportion to its concentration, and Ceratonia siliqua B exhibited a melanogenesis inhibiting effect exceeding 30% at a concentration of 100 μM.

[Table 1]

| Item | Concentration (μM) | Melanogenesis inhibition rate (%) |
|---|---|---|
| Blank control | - | 0.0±2.5 |
| L-Ascorbic acid (Positive control) | 10 | 32.8±3.4 ** |
| | 0.1 | 9.2±1.6 |
| | 5 | 15.6±1.3 |
| Ceratonia siliqua B | 10 | 20.2±3.1 * |
| | 50 | 25.6±2.6 * |
| | 100 | 30.6±3.0 * |

($\bar{x}$±S, n=3)
** : P<0.01, * : P<0.05

The present invention is not limited to the above-described embodiments or examples, and includes various modifications without deviating from the scope of the invention described in the claims.

Industrial Applicability

The novel phenylpropanoid compound of the present invention is used as a whitening agent, an melanogenesis inhibitor, an antioxidant agent, a skin external preparation and a cosmetic, and is widely used in the medical and cosmetic fields.

## Claims

**1.** A compound represented by the following formula (I) or a salt thereof, or a solvate thereof.

[Formula 1]

(I)

**2.** A whitening agent comprising a compound represented by the following formula (I) or a salt thereof, or a solvate thereof.

[Formula 2]

(I)

3. The whitening agent according to claim 2, wherein the concentration of the compound represented by the following formula (I) or a salt thereof, or a solvate thereof is 0.001 mM to 1 mM.

4. A melanogenesis inhibitor comprising a compound represented by the following formula (I) or a salt thereof, or a solvate thereof.

[Formula 3]

(I)

5. An antioxidant agent comprising a compound represented by the following formula (I) or a salt thereof, or a solvate thereof.

[Formula 4]

(I)

6. The agent according to any one of claims 2 to 5, which is a skin external preparation.

7. The agent according to any one of claims 2 to 5, which is a cosmetic.

8. A food and drink composition for skin whitening comprising a compound represented by the following formula (I) or a salt thereof, or a solvate thereof.

[Formula 5]

(I)

9. A method for producing a compound represented by the following formula (I), comprising:

obtaining a hydroalcoholic extract of pods of locust bean (*Ceratonia siliqua*);
subjecting the hydroalcoholic extract to liquid-liquid extraction with petroleum ether and ethyl acetate in this order to collect ethyl acetate fractions; and
separating the compound from the ethyl acetate fractions.

[Formula 6]

(I)

# FIG. 1

+MS, 0.3-0.6min #18-33
Intens.
x10^6
1.0
0.8
0.6
0.4
0.2
0.0
465.1983
503.2368
367.2285
415.2508
309.2200
283.1688
233.1142
588.2936
675.2978
200
300
400
500
600
700
m/z

# FIG. 2

No. Wavelength (nm.) Abs.
1 269.20 1.0639
2 211.60 2.3436
3 193.60 0.6026
1
3
2.500
2.000
1.500
1.000
0.500
0.000
Abs
190.0 200.0
250.0
300.0
350.0
400.0
Wavelength [nm.]

# FIG. 3

%T
cm-1
3393.18cm-1
2933.52cm-1
1731.29cm-1
1506.71cm-1
1464.86cm-1
1421.12cm-1
1388.81cm-1
1243.07cm-1
1158.57cm-1
1129.16cm-1
1070.11cm-1
625.20cm-1
609.78cm-1
595.24cm-1
581.17cm-1

# FIG. 4

6.76
6.63
6.60
6.32
6.31
6.30
6.30
6.29
6.28
4.89
4.88
4.85
4.72
4.71
3.86
3.79
3.79
3.77
3.77
3.68
3.67
3.43
3.41
3.31
1.18
1.17
2.00
1.01
0.97
1.04
2.01
6.03
1.03
1.08
1.00
2.05
1.08
1.08
6.05
7.5 7.0 6.5 6.0 5.5 5.0 4.5 4.0 3.5 3.0 2.5 2.0 1.5 1.0 0.5 0.0
f1 (ppm)

# FIG. 5

178.5
154.3
136.2
134.8
134.5
124.4
105.6
105.2
78.3
77.8
75.7
71.3
66.0
62.5
57.0
35.2
19.3
210 190 170 150 130 110 90 80 70 60 50 40 30 20 10 0
f1 (ppm)

# FIG. 6

0
10
20
30
40
50
60
70
80
90
100
110
120
130
140
150
160
170
180
190
f1 (ppm)
7.0 6.5 6.0 5.5 5.0 4.5 4.0 3.5 3.0 2.5 2.0 1.5 1.0 0.5
f2 (ppm)

# FIG. 7

10
20
30
40
50
60
70
80
90
100
110
120
130
140
150
160
170
180
190
f1 (ppm)

# FIG. 8

| Position | $\delta_H$ (ppm) | $\delta_C$ (ppm) | Position | $\delta_H$ (ppm) | $\delta_C$ (ppm) |
|---|---|---|---|---|---|
| 1 | - | 134.5 | 3' | - | 178.5 |
| 2, 6 | 6.76, s | 105.6 | 1" | 4.89, d (J=7.8 Hz) | 105.2 |
| 3, 5 | - | 154.3 | 2" | 3.48, m | 75.7 |
| 4 | - | 136.2 | 3" | 3.43, m | 77.8 |
| 7 | 6.61, d (J=16.2 Hz) | 134.8 | 4" | 3.42, m | 71.3 |
| 8 | 6.30, dt (J=15.6 Hz, J=6.0 Hz) | 124.4 | 5" | 3.22, m | 78.3 |
| 9 | 4.71, d, (J=6.0 Hz) | 66.0 | 6" | 3.67, dd (J=12.0 Hz, J=5.4 Hz) | 62.5 |
| 1', 4' | 1.18, d (J=7.2 Hz) | 19.3 | | 3.78, dd (J=12.0 Hz, J=2.4 Hz) | |
| 2' | 2.60, m | 35.2 | $OCH_3$-3,5 | 3.86, s | 57.0 |

# FIG. 9

HO
HO
O
HO
O
OH
O
O
O
O
HMBC

# FIG. 10

100
80
60
40
20
0
Radical scavenging rate (%)
1
5
10
50
100
[μM]

# FIG. 11

150
100
50
0
cell viability
( % of control )
Control
1
5
10
50
100
[μM]

# FIG. 12

1.5
1.0
0.5
0.0
Amount of ROS generated(× Basal)
1
5
10
50
100
Vit.C
Ceratonia siliqua B [μM]

# FIG. 13

150
100
50
0
cell viability
( % of control )
Control
1
5
10
50
100
[μM]

**INTERNATIONAL SEARCH REPORT**

International application No. **PCT/JP2022/011172**

**A. CLASSIFICATION OF SUBJECT MATTER**

***C07H 15/203***(2006.01)i; ***A23L 33/105***(2016.01)i; ***A61K 8/60***(2006.01)i; ***A61K 31/7034***(2006.01)i; ***A61K 36/48***(2006.01)i; ***A61P 17/00***(2006.01)i; ***A61P 17/18***(2006.01)i; ***A61Q 19/00***(2006.01)i; ***A61Q 19/02***(2006.01)i

FI: C07H15/203 CSP; A61Q19/02; A61K8/60; A61P17/18; A61P17/00; A61K31/7034; A61K36/48; A61Q19/00; A23L33/105

According to International Patent Classification (IPC) or to both national classification and IPC

**B. FIELDS SEARCHED**

Minimum documentation searched (classification system followed by classification symbols)

C07H15/203; A23L33/105; A61K8/60; A61K31/7034; A61K36/48; A61P17/00; A61P17/18; A61Q19/00; A61Q19/02

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X | KR 10-2006-0093164 A (LG HOUSEHOLD & HEALTH CARE LTD.) 24 August 2006 (2006-08-24)<br>claims, p. 3, paragraph 2, examples | 2-3, 6-7 |
| A | JP 2010-504299 A (CHANEL PARFUMS BEAUTE) 12 February 2010 (2010-02-12)<br>entire text | 1-9 |
| A | JP 7-61915 A (SANSHO PHARMACEUTICAL CO., LTD.) 07 March 1995 (1995-03-07)<br>entire text | 1-9 |
| A | ONO, M. et al., Antioxidative and Antihyaluronidase Activities of Some Constituents from Foeniculi Fructus (Fruit of Foeniculum vulgare MILLER), Food Science and Technology International, Tokyo, 1997, vol. 3, no. 1, pp. 53-55, DOI 10.3136/fsti9596t9798.3.53<br>entire text | 1-9 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **10 May 2022** | **24 May 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**
**Information on patent family members**

International application No.
**PCT/JP2022/011172**

| Patent document cited in search report | Publication date (day/month/year) | Patent family member(s) | Publication date (day/month/year) |
|---|---|---|---|
| KR 10-2006-0093164 A | 24 August 2006 | (Family: none) | |
| JP 2010-504299 A | 12 February 2010 | US 2010/0028471 A1 | |
| | | WO 2008/034821 A1 | |
| | | EP 2063860 A1 | |
| JP 7-61915 A | 07 March 1995 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2005119999 A


### Non-patent literature cited in the description


- **RTIBI K ; JABRI M A ; SELMI S et al.** Preventive effect of carob (Ceratonia siliqua L.) in dextran sulfate sodium-induced ulcerative colitis in rat. *RSC Advances,* 2016, vol. 6, 19992-20000
- **RTIBI K ; SELMI S ; GRAMI D et al.** Chemical constituents and pharmacological actions of carob pods and leaves (Ceratonia siliqua L.) on the gastrointestinal tract: A review. *Biomedicine & Pharmacotherapy,* 2017, vol. 93, 522-528